# EUROPEAN PATENT SPECIFICATION

(11) **EP 0 775 505 B1**
(45) Date of publication and mention of the grant of the patent: **01.09.2004**
(21) Application number: 95308296.3
(22) Date of filing: 21.11.1995
(51) Int. Cl.: A61N 5/10

(54) **A minimally invasive medical device for providing a radiation treatment**
Minimal-invasives medizinisches Gerät für Strahlentherapie
Dispositif médical agressif de façon minimale fournissant un traitement à base de radiations

(43) Date of publication of application: 28.05.1997
(73) Proprietor: MED INSTITUTE, INC., West Lafayette Indiana 47906 (US)
(72) Inventor: Fearnot, Neal E., West Lafayette, IN 47906 (US)
(74) Representative: Johnston, Kenneth Graham

(56) References cited:
- EP-A- 0 633 041
- DE-U- 9 102 312

## Description

This invention relates to minimally invasive medical devices.

Occlusion of vessels such as coronary arteries, femoral or iliac vessels, can be repaired by minimally invasive procedures such as balloon angioplasty or laser ablation. During these procedures abrasion or dissection of the vessel wall may occur during reopening or enlargement and thrombi formation and occlusion may occur.

Alternatively, a stent may be employed to maintain the patency of the vessel. A problem with the use of a stent is that smooth muscle proliferates or intimal hyperplasia tends to occur within a period of six months.

According to the present invention, there is provided medical apparatus as defined in claim 1. The apparatus provides a controlled radiation treatment to a blood vessel.

The expandable distal portion includes spring wires looped about a distal end of the inner elongated member for expanding and contacting the wall of the blood vessel when extended from the distal end of the outer sheath. A similar device is known from EP-A-0 633 041 (embodiment of Fig.6), in which a radioactive filament is coiled around a guide wire. According to the invention, however, the radiation source comprises a radioactive material such as iridium and the like, which in the preferred embodiment is combined with the spring wires to form radioactive metal alloy spring wires. In another aspect, the radiation source forms a radioactive surface coating or layer disposed on the outer surface of the spring wires. In yet another aspect, the radiation source comprises radioactive material sleeves attached to the spring wires.

In another aspect of the invention, the medical device includes a wire guide extendable through the distal portion of the expandable spring wire arrangement or frame. To advantageously affect a greater mass of radioactive material, the spring wires are helically shaped. The medical device also includes a coil extending distally from and rotatable with respect to the spring wire basket. To minimize trauma to the vessel wall, the coil has a distal end with a greater flexibility than that of the proximal end and further includes a tapered mandril positioned longitudinally within the coil.

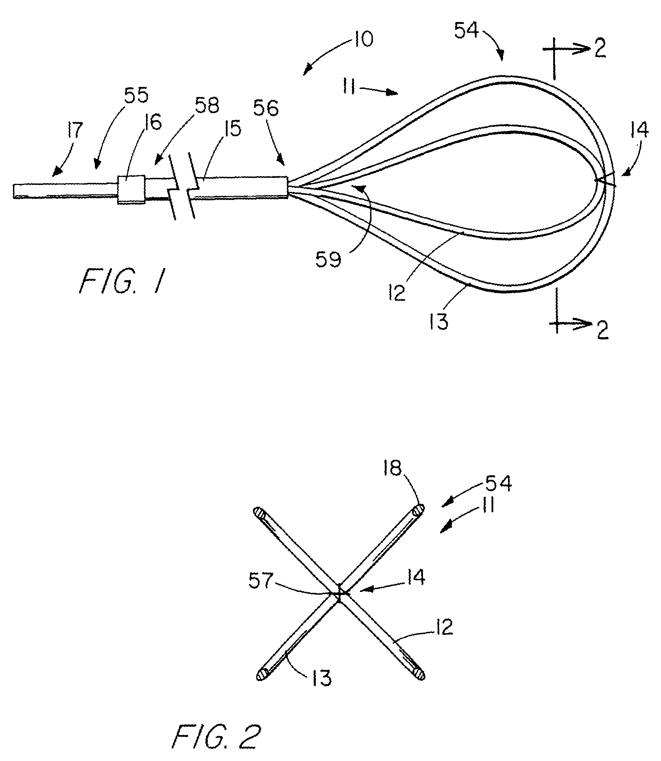

The radiation dosage is preferably selected to be of a magnitude such that within a reasonable exposure time, for example 5 to 10 minutes or possibly even longer, the exposure of the wall of the vessel is sufficient that the wall moves away from the enlargement means, i.e., a wire frame as shown in FIGs. 1 to 10. Furthermore, the wall is expanded further by the radiation exposure. The strength of the radiation is thus predetermined.

### Brief Description of the Drawing

FIG. 1 depicts a preferred embodiment of the minimally invasive medical device of the present invention;
FIG. 2 depicts an enlarged cross-sectional view of the medical device of FIG. 1 taken along the line 2-2;
FIGs. 3-5 depict the medical device of FIG. 1 variously positioned in a coronary artery; and
FIGs. 6-10 depict various other aspects of the minimally invasive medical device of the present invention.

### Detailed Description

Depicted in FIG. 1 is a preferred embodiment of a minimally invasive medical device 10 including an expandable wire arrangement 54, for providing a selective and controlled radiation treatment in a body passageway. This minimally invasive medical device has particular application as a percutaneously inserted intravascular device for controllably providing a therapeutic radiation dosage to an affected area of a coronary vessel that is deemed likely to experience restenosis. Restenosis typically occurs after a procedure to open or enlarge the vessel, such as balloon angioplasty, laser ablation, or stent placement.

Device 10 includes an outer sheath or tube 15 with inner elongated member 55, which is longitudinally and slidably disposed through passage 59 of the outer sheath or tube. Inner elongated member includes an expandable distal portion 11 such as the expandable wire arrangement 54, such as preferably a stranded wire basket, which in an expanded condition extends from distal end 56 of the outer sheath. The stranded wire basket is formed from commercially available spring wires 12 and 13 of, for example, stainless steel, platinum or tantalum, that are looped about distal end 14 of the inner elongated member. The expandable wire arrangements 54 can alternatively be provided with central rod or wire attached to the distal end of the arrangement. The rod can be pulled or pushed relative to the arrangement 54 in order to expand or contract the arrangement. Wires 12 and 13 would not have to be of spring material when the rod is provided.

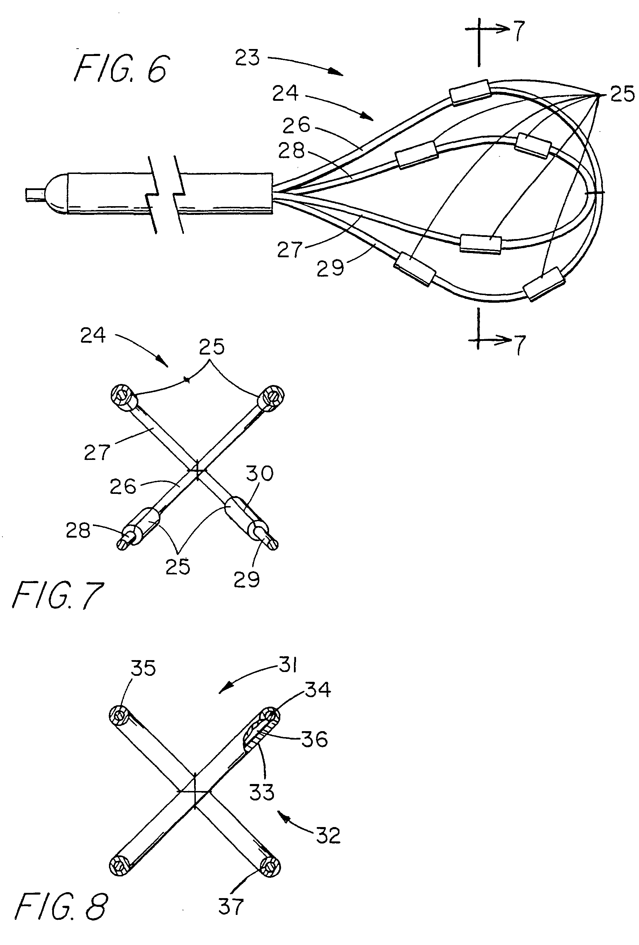

Device 10 further includes a radiation source 18 such as commercially available radioactive material, for example, iridium, which is combined with spring wires 12 and 13 to form radioactive metal alloy spring wires. Alternatively, a commercially available radioactive material surface coating 33 or outer layer is disposed about outer surfaces 36 and 37 of respective spring wires 34 and 35, as depicted in FIG. 8. Spring wires 12 and 13 extend proximally from the expandable wire basket portion through end cap 16 and outer sheath 15, which is formed from a commercially available polytetrafluoroethylene tube. Proximal end 17 of the inner elongated member or spring wires would extend beyond the proximal end of the sheath to provide a convenient grip or handle for extending and withdrawing the expandable wire basket from and into the outer sheath.

FIG. 2 depicts an enlarged cross-sectional view of expanded wire arrangement, frame, or basket 54 of FIG. 1 taken along the line 2-2. Spring wires 12 and 13 are looped at distal end 14 of the inner elongated member and affixed to one another using, for example, suture material 57 or a suitable medical grade adhesive. In a preferred embodiment, radiation source 18 is commercially available iridium which has been alloyed with stainless steel to form spring wires 12 and 13.

When device 10 is introduced into the vascular system of a patient, it is, for example, approximately 95 cm in length and 8 French (2.7 mm or .105") in diameter when the expandable distal portion is collapsed and retracted within the outer sheath. Expandable distal portion 11 is approximately 7 mm long and 3 mm in diameter when extended from the sheath to assume an open, expanded condition that allows the device to center itself in the vessel lumen. To accommodate coronary arteries, the maximum outside diameter of the expandable distal portion when in a fully expanded condition preferably falls in a range of 0.5 to 4.0 mm. However, the lengths and diameters of device 10 and expandable distal portion 11 can be adjusted to accommodate large or small blood vessels or any other body passageways desired to be irradiated. The word "expandable" means either self-expanding or being capable of expansion by other means such as the rod.

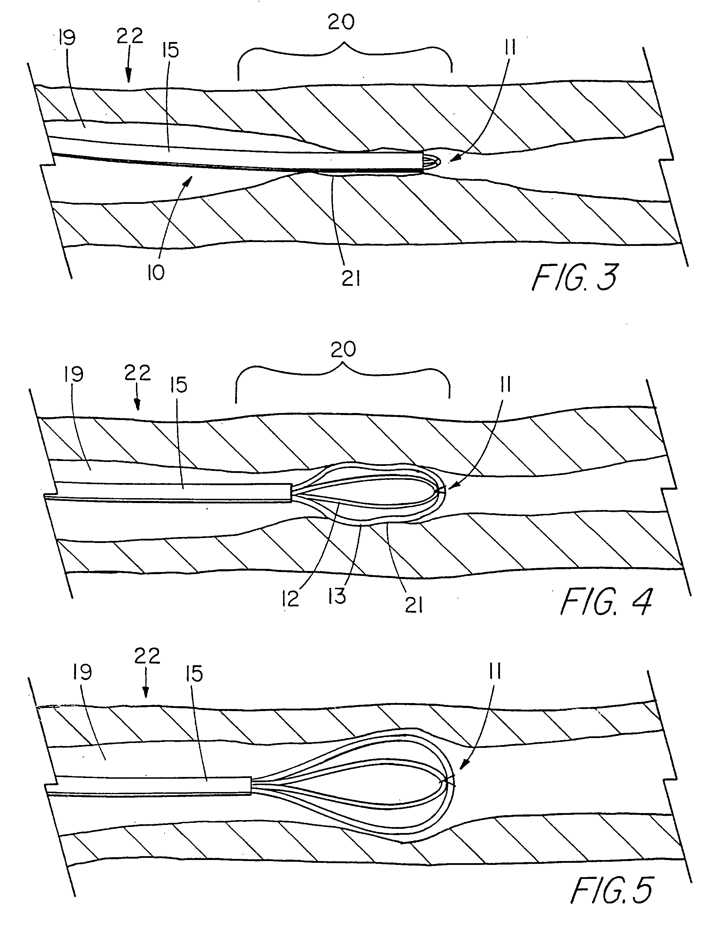

FIGs. 3-5 depict coronary artery 22 with artery lumen 19 partially occluded by intimal hyperplasia and smooth muscle cell proliferation at vessel region or treatment site 20. FIG. 3 depicts device 10 positioned in the lumen of the coronary artery with expandable distal portion 11 in the partially occluded treatment site for providing short-term, localized irradiation of the coronary artery wall. When sheath or tube 15 is pulled proximally as depicted in FIG. 4, expandable distal arrangement or frame portion 11 extends therefrom or is extended by a rod, and opens to an expanded condition. Wires 12 and 13, which can include a radioactive metal alloy, atraumatically rest against the inner surface, or intimal layer, of coronary artery wall 21 and the proliferation of cells thereon. As a result, the spring wires are positioned away from the center of the artery lumen for minimizing the interruption of blood flow therethrough.

FIG. 5 depicts artery 22 after a short-term, localized radiation treatment with lumen 19 widened beyond the extent of the wires about the treatment site. The spring wires of the expandable frame portion are no longer in contact with the artery wall. It is to be noted that with this form of apparatus, the radiation of the wall of the artery has caused the latter to expand beyond the environs of the expanded distal portion. To remove device 10 from the coronary artery, sheath or tube 15 is pushed distally over expandable distal basket portion 11 for collapsing the spring wires and containing them in the sheath. Collapse could alternatively be achieved by initially pushing on the central rod to collapse the cage and then to pull on the rod and the elongated member so that they are confined within sheath 15.

FIG. 6 depicts a minimally invasive medical device 23, which includes an expandable wire arrangement such as a basket 24 similar to device 10 except for the radiation source. The radiation source includes a plurality of commercially available radioactive iridium material tubular sleeves 25 disposed around outer surfaces 26 and 27 of spring wires 28 and 29 using soft solder 30, as depicted in FIG. 7. FIG. 7 is an enlarged cross-sectional view of expandable arrangement 24 of FIG. 6 taken along the line 7-7. Alternatively, the plurality of radioactive material tubular sleeves 25 is affixed about the outer surface of the spring wires by crimping.

FIG. 8 depicts minimally invasive medical device 31, which includes expandable distal portion or arrangement 32 and the radiation source in the form of a radioactive surface coating 33 or a radioactive outer layer disposed on outer surfaces 36 and 37 of respective spring wires 34 and 35. The surface coating includes a plastic material with radioactive iridium particles dispersed throughout. The outer layer includes radioactive iridium plated on the spring wires.

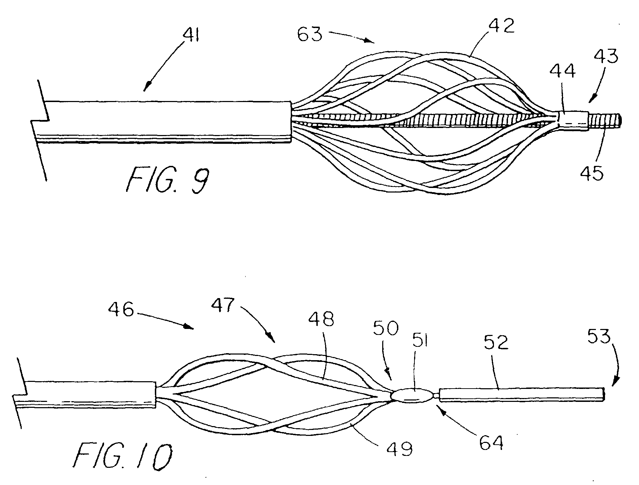

FIG. 9 depicts a minimally invasive medical device or apparatus 41, which represents yet another embodiment of the invention. Device 41 includes a plurality (eight) of interweaved wires 42 attached about distal end 43 of an inner elongated member if the wires are of spring material, then the distal end 43 of the wires can be soldered (44) to the inner rod 45 or guide. If the wires are not of spring material, they can be expanded by moving proximally the end 43 to the end of the sleeve thereby expanding the wires. The wire guide 45 is depicted extending through device 41 for facilitating advancement of the device through the vascular system and to the treatment site of a patient. Radioactivity of the wall of the vessel is provided by making guide 45 of radioactive material at least partially, and by providing radioactive material in one or more of wires 42 in a similar manner to the previous embodiments.

FIG. 10 depicts minimally invasive medical device 46, which represents yet another embodiment of the invention. The device includes expandable wire portion 47 and radiation source 48 in the form of a surface coating or an outer layer disposed thereon. Expandable distal portion 47 includes a plurality (four) of helically positioned wires 49 attached about distal end 50 using, for example, soft solder. Device 46 further includes swivel connection 51 extending between the plurality of four helical wires 49 and flexible wire guide coil 52. The flexible coil provides a deflectable, atraumatic means for maintaining the position of the wire basket in a body passageway during a therapeutic procedure. The flexible wire guide coil is positioned over a tapered mandril or, alternatively, a straight length of round wire and a safety wire, so that flexible wire guide coil 52 exhibits a gradual increase in flexibility toward distal end 53 thereof. Swivel connection 51 provides for the rotation of expandable basket portion 46 while flexible coil 52 remains stationary.

It is to be understood that the above-described minimally invasive intravascular medical device for providing a radiation treatment in a body passageway is merely an illustrative embodiment of the principles of this invention and that other devices, instruments, or apparatus may be devised. In particular, it is contemplated that a radioactive material other than an iridium material may be used as a radiation source. It is further contemplated that many commercially available radially expandable medical arrangements may be provided to attach a radiation source about the expandable portion thereof for providing the radiation treatment to a body passageway and, in particular, preventing intimal hyperplasia and smooth muscle proliferation which causes stenosis or restenosis of a blood vessel passageway. Although described as being particularly applicable to the vascular system using percutaneous insertion techniques, it is contemplated that the minimally invasive device is applicable for providing treatment to the pulmonary system as well as the gastrointestinal tract. Treatment of the biliary and urinary system are also contemplated with expandable medical devices such as wire baskets, particularly adapted with the radiation source for treating the particular anatomical system.

In each of the embodiments described, the radiation means is part of the expandable means such as frames that are used to expand the wall of the vessel, and as long as the expandable means stays in position, the radiation also stays in position and consequently radiates the wall throughout the expansion period.

## Claims

1. A minimally invasive medical apparatus (10) for providing radiation treatment to a wall of a passage in a patient, said apparatus comprising:
a tube or outer sheath (15) having a distal end (56), a proximal end of the outer sheath, and a passage (59) extending longitudinally therein;
an expandable spring arrangement (54, 24, 32, 47) comprising spring wires (12, 13, 26, 27, 28, 29, 34, 35, 42, 49) for expanding the wall ofthe passage in the patient,
a radioactive material, and an inner elongated member (55), having an expandable distal portion (11) comprising the said expandable spring arrangement and a proximal portion (17), the inner elongated member being slidably disposed relative to said passage (59), said proximal portion (17) extending from the proximal end of the outer sheath (15) for extending the expandable distal portion (11) from within the outer sheath at the distal end thereof, and withdrawing the expandable distal portion (11) to within the outer sheath at said distal end, the expandable distal portion (11) having an expanded condition when extended from the distal end of the outer sheath (15) in order to expand the wall of the passage in the patient, **characterised in that** the radioactive material is combined with the spring wires to form radioactive metal alloy spring wires or forms a radioactive surface coating or layer disposed on the outer surface of the spring wires or comprises radioactive material sleeves attached to the spring wires, in order to contact and irradiate the expanded wall of the passage in the patient.

2. Apparatus of claim 1, wherein said expandable spring arrangement (54, 42, 47) of the distal portion includes a plurality of loops of wire (12, 13, 26, 28, 27, 29, 34, 35, 42, 49).

3. Apparatus of claim 1, wherein the radioactive material is provided by sleeves (25) disposed around said wires (12, 13, 26, 28, 27, 29, 34, 35, 42, 49) of the expandable spring arrangement (54, 42, 47).

4. Apparatus of any one of claims 1, 2 or 3 wherein the wires (12, 13, 26, 28, 27, 29, 34, 35, 42, 49) of the expandable spring arrangement (54, 42, 47) are spring wires and for example are helically shaped.

5. Apparatus of claim 4, further comprising a coil (52) extending distally from and rotatably connected to said spring wires (47), the distal end (53) of the coil (52) optionally having a greater flexibility than that of said proximal end.

6. Apparatus of claim 5 wherein said coil (52) includes a tapered mandril positioned longitudinally in said coil.

7. Apparatus of any one of claims 1 to 4, wherein an inner rod or wire (45) extends from within the distal end of the outer sheath (41, 56) to the distal end (43) of the inner elongated member (55) and wherein the inner rod or wire (45) is at least partially of radioactive material.

## Patentansprüche

1. Minimal-invasives medizinisches Gerät (10) für die Strahlentherapie einer Wand eines Durchgangs in einem Patienten, wobei das Gerät Folgendes umfasst: eine Röhre oder Außenschleuse (15) mit einem distalen Ende (56), einem proximalen Ende der Außenschleuse und einem Durchgang (59), der sich in Längsrichtung darin erstreckt; eine expandierbare Federanordnung (54, 24, 32, 47) mit Federdrähten (12, 13, 26, 27, 28, 29, 34, 35, 42, 49) zum Erweitern der Wand des Durchgangs im Patienten, ein radioaktives Material und ein inneres längliches Element (55) mit einem expandierbaren distalen Teil (11), der die expandierbare Federanordnung umfasst, und einem proximalen Teil (17), wobei das innere längliche Element relativ zum Durchgang (59) verschieblich angeordnet ist, wobei der proximale Teil (17) sich vom proximalen Ende der Außenschleuse (15) zum Verlängern des expandierbaren distalen Teils (11) vom Inneren der Außenschleuse an ihrem distalen Ende und zum Zurückziehen des expandierbaren distalen Teils (11) in das Innere der Außenschleuse am distalen Ende erstreckt, wobei der expandierbare distale Teil (11) einen erweiterten Zustand aufweist, wenn er vom distalen Ende der Außenschleuse (15) zum Erweitern der Wand des Durchgangs im Patienten verlängert wird, **dadurch gekennzeichnet, dass** das radioaktive Material zur Bildung von Federdrähten aus einer radioaktiven Metalllegierung mit Federdrähten kombiniert ist oder eine radioaktive Oberflächenbeschichtung oder eine Schicht auf der Außenseite der Federdrähte bildet oder radioaktive Materialhüllen umfasst, die an den Federdrähten befestigt sind, um die erweiterte Wand des Durchgangs im Patienten zu kontaktieren und zu bestrahlen.

2. Gerät nach Anspruch 1, worin die expandierbare Federanordnung (54, 42, 47) des distalen Teils eine Vielzahl von Drahtschlaufen (12, 13, 26, 28, 27, 29, 34, 35, 42, 49) enthält.

3. Gerät nach Anspruch 1, worin das radioaktive Material von Hüllen (25) geliefert wird, die um die Drähte (12, 13, 26, 28, 27, 29, 34, 35, 42, 49) der expandierbaren Federanordnung (54, 42, 47) herum angeordnet sind.

4. Gerät nach einem der Ansprüche 1, 2 oder 3, worin die Drähte (12, 13, 26, 28, 27, 29, 34, 35, 42, 49) der expandierbaren Federanordnung (54, 42, 47) Federdrähte sind und beispielsweise spiralförmig sind.

5. Gerät nach Anspruch 4, ferner umfassend eine Spule (52), die sich distal von den Federdrähten (47) aus erstreckt und drehbar mit diesen verbunden ist, wobei das distale Ende (53) der Spule (52) optional eine größere Flexibilität aufweist als das proximale Ende.

6. Gerät nach Anspruch 5, worin die Spule (52) einen verjüngten Mandrin aufweist, der in Längsrichtung in der Spule angeordnet ist.

7. Gerät nach einem der Ansprüche 1 bis 4, worin sich eine Innenstange oder ein Draht (45) vom Inneren des distalen Endes der Außenschleuse (41, 56) bis zum distalen Ende (43) des inneren länglichen Elements (55) erstreckt und worin die Innenstange oder der Draht (45) zumindest teilweise aus radioaktivem Material besteht.

## Revendications

1. Appareil médical minimalement effractif (10) destiné à soumettre à une radiothérapie une paroi d'un passage chez un patient, ledit appareil comprenant :
un tube ou une gaine externe (15) comportant une extrémité distale (56), une extrémité proximale de la gaine externe, et un passage (59) se prolongeant longitudinalement dedans ;
un arrangement à ressort dilatable (54, 24, 32, 47) comprenant des fils à ressort (12, 13, 26, 27, 28, 29, 34, 35, 42, 49) pour la dilatation de la paroi du passage chez le patient ;
une matière radioactive ; et
un élément allongé interne (55), comportant une partie distale dilatable (11) comprenant ledit arrangement à ressort dilatable et une partie proximale (17), l'élément allongé interne étant disposé de façon coulissante par rapport audit passage (59), ladite partie proximale (17) se prolongeant à partir de l'extrémité proximale de la gaine externe (15) pour l'allongement de la partie distale dilatable (11) à partir de l'intérieur de la gaine externe à l'extrémité distale de celle-ci, et la rétraction de la partie distale dilatable (11) à l'intérieur de la gaine externe à ladite extrémité distale, la partie distale dilatable (11) se trouvant dans un état dilaté quand elle est allongée à partir de l'extrémité distale de la gaine externe (15) pour dilater la paroi du passage chez le patient, **caractérisé en ce que** la matière radioactive est combinée avec les fils à ressort pour former des fils à ressort en alliage métallique radioactif, ou forme un revêtement de surface radioactif ou une couche radioactive disposé(e) sur la surface externe des fils à ressort, ou forme des manchons en matière radioactive fixés aux fils à ressort, afin d'entrer en contact avec la paroi dilatée du passage chez le patient et d'irradier cette paroi.

2. Appareil selon la revendication 1, dans lequel ledit arrangement à ressort dilatable (54, 42, 47) de la partie distale comporte une pluralité de boucles de fil (12, 13, 26, 28, 27, 29, 34, 35, 42, 49).

3. Appareil selon la revendication 1, dans lequel la matière radioactive est apportée par des manchons (25) disposés autour desdits fils (12, 13, 26, 28, 27, 29, 34, 35, 42, 49) de l'arrangement à ressort dilatable (54, 42, 47).

4. Appareil selon l'une quelconque des revendications 1, 2 ou 3, dans lequel les fils (12, 13, 26, 28, 27, 29, 34, 35, 42, 49) de l'arrangement à ressort dilatable (54, 42, 47) sont des fils à ressort et sont par exemple de forme hélicoïdale.

5. Appareil selon la revendication 4, comprenant en outre une pièce enroulée (52) qui se prolonge distalement à partir desdits fils à ressort (47) et qui est raccordée de façon pivotante à ceux-ci, l'extrémité distale (53) de la pièce enroulée (52) ayant éventuellement une plus grande flexibilité que ladite extrémité proximale.

6. Appareil selon la revendication 5, dans lequel ladite pièce enroulée (52) comporte un mandrin conique positionné longitudinalement dans ladite pièce enroulée.

7. Appareil selon l'une quelconque des revendications 1 à 4, dans lequel une tige ou un fil interne (45) se prolonge à partir de l'intérieur de l'extrémité distale de la gaine externe (41, 56) jusqu'à l'extrémité distale (43) de l'élément allongé interne (55) et dans lequel la tige ou le fil interne (45) est constitué (e) au moins en partie de matière radioactive.
